# EUROPÄISCHE PATENTANMELDUNG

(11) **EP 3 466 353 A1**
(43) Veröffentlichungstag der Anmeldung: **10.04.2019**
(21) Anmeldenummer: 18198446.9
(22) Anmeldetag: 03.10.2018
(51) Int. Cl.: A61B 17/28

(54) **MEDIZINISCHE KLEMME**

(30) Priorität: 04.10.2017 AT 508482017
(71) Anmelder: Stoiber, Friedrich, 4632 Pichl (AT)
(72) Erfinder: Stoiber, Friedrich, 4632 Pichl (AT)
(74) Vertreter: Patentanwaltskanzlei Hübscher

(57) **Zusammenfassung**

Es wird eine medizinische Klemme (1) mit zwei um eine Anlenkachse (2) drehbar miteinander verbundenen, gekreuzten Armen (3), die je einen Griffteil (4) und ein dem Griffteil (4) gegenüberliegendes Maulteil (5) aufweisen, beschrieben. Um einfache medizinische Eingriffe mit nur einem medizinischen Halteinstrument vornehmen zu können, ohne dass dabei die Gefahr besteht, umliegende Gewebeabschnitte zu verletzen oder zu beschädigen, wird vorgeschlagen, dass die Maulteile (5) je zwei in Richtung der Anlenkachse (2) voneinander beabstandete Klemmabschnitte (6) aufweisen.

## Beschreibung

Die Erfindung bezieht sich auf eine medizinische Klemme mit zwei um eine Anlenkachse drehbar miteinander verbundenen, gekreuzten Armen, die je einen Griffteil und ein dem Griffteil gegenüberliegendes Maulteil aufweisen.

Es sind eine Vielzahl von medizinischen Klemmen für medizinische Zwecke, insbesondere Ligaturklemmen bekannt, die Gewebe halten oder klemmen, ohne es zu verletzen (DE 459916 A, DE 929750 A).

Hierzu wurde bereits vorgeschlagen (DE 1060546 B), an den Klemmabschnitten Längsrippen und Längsnuten anzuordnen, die ein atraumatisches Halten bzw. Klemmen von Gewebe ermöglichen. Nachteilig ist daran allerdings, dass mit solchen Klemmen üblicherweise ein Gewebe- oder Gefäßabschnitt nicht derart präpariert werden kann, dass eine Behandlung ohne zusätzliche Haltemaßnahmen oder die Gefahr einer Verletzung des umliegenden Gewebes ermöglicht wird.

Aus dem Stand der Technik sind darüber hinaus Retraktoren bekannt (DE 2951664 A1), die das Aufspannen von Gewebeöffnungen ermöglichen, um darunterliegendes Gewebe freizulegen und behandeln zu können. Derartige Retraktoren bringen allerdings den Nachteil mit sich, dass die zu behandelnden Gewebeteile nicht gehalten bzw. festgeklemmt werden können, sodass stets mehrere medizinische Halteinstrumente erforderlich sind, um einen Eingriff vorzunehmen.

Der Erfindung liegt somit die Aufgabe zugrunde, eine medizinische Klemme der eingangs beschriebenen Art so auszugestalten, dass einfache medizinische Eingriffe mit nur einem medizinischen Halteinstrument vorgenommen werden können, ohne dass dabei die Gefahr besteht, umliegende Gewebeabschnitte zu verletzen oder zu beschädigen.

Die Erfindung löst die gestellte Aufgabe dadurch, dass die Maulteile je zwei in Richtung der Anlenkachse voneinander beabstandete Klemmabschnitte aufweisen.

Zufolge dieser Maßnahmen wird dem Anwender sowohl ein Ergreifen des zu behandelnden Gewebeteils, als auch ein gleichmäßiges Aufspannen des Gewebeteils in Richtung der Anlenkachse zwischen den Klemmabschnitten ermöglicht. Der zu behandelnde Gewebeteil kommt somit im freibleibenden Zwischenraum zwischen den beiden gabelförmig angeordneten Klemmabschnitten zu liegen und kann dort behandelt werden, während die Positionierung der erfindungsgemäßen Klemme sicherstellt, dass kein umliegendes Gewebe verletzt oder gar beschädigt wird. Besonders einfache Konstruktionsbedingungen ergeben sich in diesem Zusammenhang, wenn die beiden Maulteile U-förmig ausgebildet sind und über deren Basisschenkel, an den die beiden Klemmabschnitte in Richtung der Längsachse des jeweils zugeordneten Armes auf der der Anlenkachse gegenüberliegenden Seite anschließen, mit dem jeweils zugeordneten Arm verbunden sind. Grundsätzlich kann die Form des Basisschenkels frei gewählt werden, die Gefahr einer Verletzung umliegenden Gewebes kann aber insbesondere dann vermieden werden, wenn der Basisschenkel zu den Klemmabschnitten hin eine Krümmung aufweist, sodass die Bildung von Kanten vermieden wird. Der Basisschenkel kann zu diesem Zweck beispielsweise auch V-förmig ausgebildet werden. Der Abstand der beiden Klemmabschnitte zueinander kann je nach Anwendungsgebiet unterschiedlich ausfallen, wobei sich vorteilhafte Handhabungsbedingungen bei einem Verhältnis des Abstandes zweier Klemmabschnitte eines Maulteiles zur Länge eines Klemmabschnittes von 5:12, 1:2 oder 3:4 ergeben haben.

Die Klemmabschnitte können in Schließrichtung atraumatische, zueinander versetzte und in Längsrichtung der Klemmabschnitte verlaufende Klemmflächen aufweisen, damit Gewebeteile, die zwischen diesen Klemmabschnitten gehalten oder eingeklemmt werden, nicht beschädigt respektive traumatisiert werden. Insbesondere können die zueinander versetzten und in Längsrichtung der Klemmabschnitte verlaufenden Klemmflächen zwei miteinander zusammenwirkende Wellenprofile bilden, die für Ihre atraumatische Wirkung für den Fachmann bekannt sind.

Um das unbeabsichtigte Eindringen von umliegendem Gewebe in den sich zwischen den beiden Klemmabschnitten ergebenden Behandlungsraum zu vermeiden, was die Gefahr einer Verletzung dieses umliegenden Gewebes mit sich bringen würde, wird vorgeschlagen, dass wenigstens an einen Klemmabschnitt in Öffnungsrichtung der Klemme eine normal zur Anlenkachse verlaufende Rückhaltefläche ansetzt. Diese Rückhaltefläche dient der Abstützung des umliegenden Gewebes, wobei deren Anordnung insbesondere dann von Vorteil ist, wenn sich der zu behandelnde Gewebeteile nahe an einem umliegenden Gewebeabschnitt befindet, sodass dessen unmittelbare Beeinträchtigung zu befürchten ist. Überdies ermöglichen die Rückhalteflächen dem Anwender, sich mit der Klemme an einem umliegenden Gewebeabschnitt abzustützen ohne dieses zu verletzen, was in einer ruhigeren Handführung und somit einem besseren Behandlungsergebnis resultiert. Gute Handhabungsbedingungen ergeben sich für die erfindungsgemäße Klemme, wenn das Verhältnis der vom jeweiligen Klemmabschnitt zu dem dem Klemmabschnitt gegenüberliegenden Endabschnitt der Rückhaltefläche gemessenen Breite des Klemmabschnittes zu dessen Länge 9:19, 5:8 oder 5:7 beträgt.

In diesem Zusammenhang hat es sich als besonders vorteilhaft erwiesen, wenn an zwei einander gegenüberliegende Klemmabschnitten beider Maulteile jeweils in Öffnungsrichtung der Klemme eine normal zur Anlenkachse verlaufende Rückhaltefläche ansetzt. Im Klemmstellung ergeben beide Rückhalteflächen folglich eine Art Schild, der das zu behandelnde zwischen den Klemmabschnitten aufgespannte Gewebeteil vollständig gegenüber dem umliegenden Gewebe einer Seite der Klemme abschirmt.

Gemäß einer weiteren Ausführungsform kann wenigstens eine der Rückhalteflächen auf der dem Klemmabschnitt gegenüberliegenden Seite einen ovalen Endabschnitt aufweisen. Gemeinsam mit den oben genannten Merkmalen kann dadurch eine Verletzung des umliegenden und durch die Rückhalteflächen vom Behandlungsbereich ferngehaltenen Gewebes zusätzlich vermieden werden, weil dadurch die Bildung von spitzen Ecken oder Kannten verhindert wird.

Um eine punktuelle Druckbelastung des umliegenden Gewebes zu vermeiden, kann der wenigstens eine Klemmabschnitt eines Maulteiles auf seiner dem zweiten Klemmabschnitt dieses Maulteiles gegenüberliegenden Seite bündig mit der Rückhaltefläche abschließen. Dabei ist unerheblich, ob die Rückhalteflächen mit den Klemmabschnitten verschweißt oder zusammen aus einem Guss gefertigt werden.

Die erfindungsgemäße Klemme kann im Klemmstellung durch zwei gegenläufige Zahn- oder Sperrstangen, die jeweils an beiden Griffteilen ausgebildet sind, in Ihrer Position fixiert werden, wodurch wiederum verbesserte Behandlungsbedingungen erreicht werden.

In der Zeichnung ist der Erfindungsgegenstand beispielsweise dargestellt. Es zeigen
- Fig. 1: eine perspektivische Ansicht einer ersten Ausführungsform der erfindungsgemäßen medizinischen Klemme,
- Fig. 2: eine Draufsicht dieser Ausführungsform in einem größeren Maßstab, die
- Fig. 3: eine Vorderansicht dieser Ausführungsform in einem noch größeren Maßstab,
- Fig 4: eine perspektivische Ansicht einer zweiten Ausführungsform der erfindungsgemäßen medizinischen Klemme,
- Fig 5: eine Draufsicht dieser Ausführungsform in einem größeren Maßstab und
- Fig 6: eine Vorderansicht dieser Ausführungsform in einem noch größeren Maßstab.

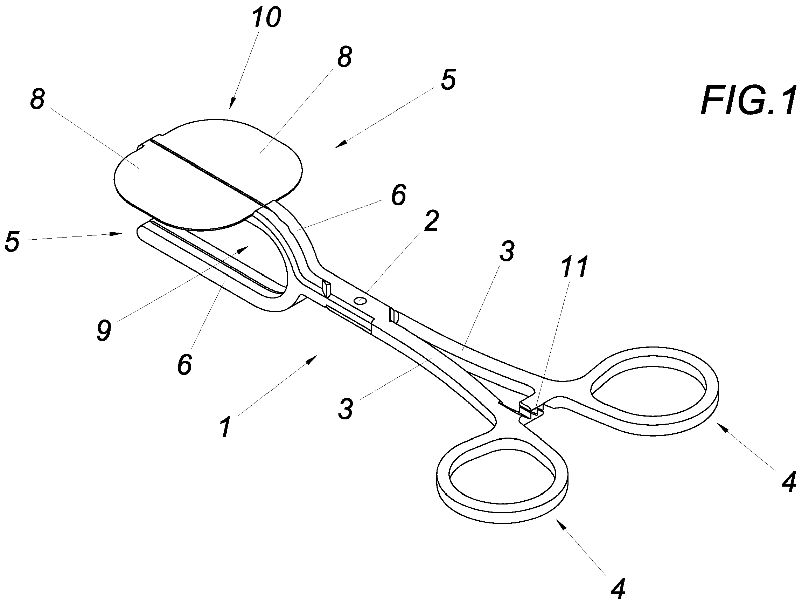

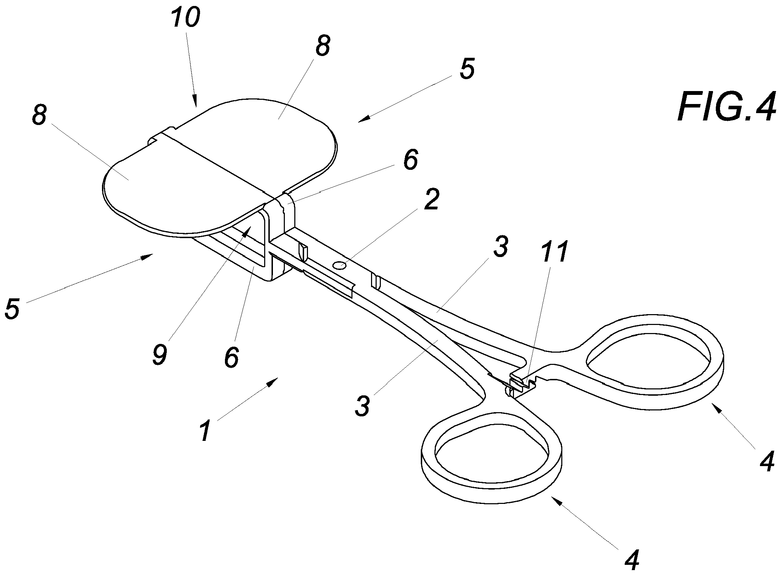

Eine erfindungsgemäße medizinische Klemme 1 weist zwei um eine Anlenkachse 2 drehbar miteinander verbundene und gekreuzte Arme 3 auf. Dabei weisen die Arme 3 je einen Griffteil 4 und ein dem Griffteil 4 gegenüberliegendes Maulteil 5 auf, wobei die Griffteile 4 zur Aufnahme von Daumen und Zeigefinger bzw. Daumen und Ringfinger ausgebildet sind und die Maulteile 5 je zwei in Richtung der Anlenkachse 2 voneinander beabstandete Klemmabschnitte 6 aufweisen. Über die Griffteile 4 können die Maulteile 5 zwischen einer Offenstellung zum Fassen von Gewebeteilen und einer, in den Figs. 1 - 6 dargestellten Klemmstellung hin- und her bewegt werden.

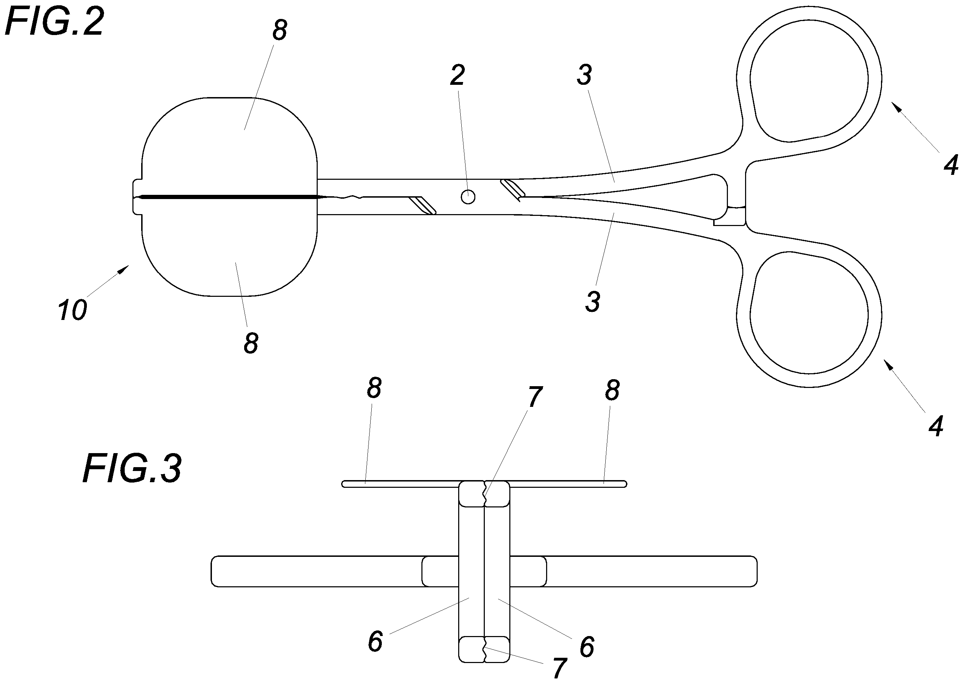

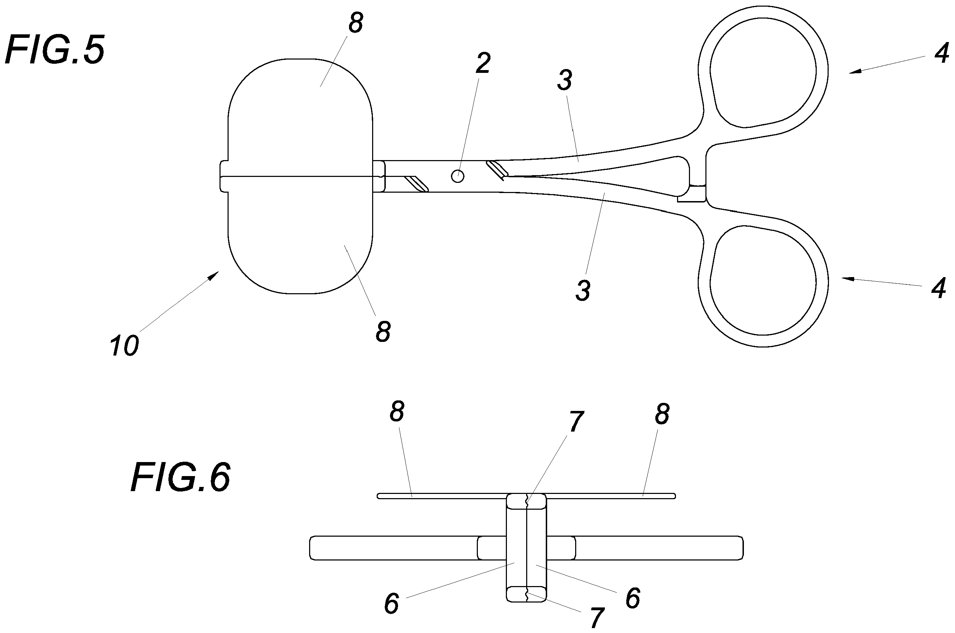

Die Klemmabschnitte 6 besitzen zueinander versetzte und in Längsrichtung der Klemmabschnitte 6 verlaufende Klemmflächen 7, welche zwei miteinander zusammenwirkende Wellenprofile bilden, wie dies insbesondere in den Figs. 3 und 6 dargestellt ist. Durch diese atraumatischen Klemmflächen 7 wird eine Schädigung des festgehaltenen Gewebes während der Behandlung in Klemmstellung der medizinischen Klemme 1 vermieden.

Um das zu behandelnde Gewebeteil gut von umliegendem Gewebe zu isolieren und so den Behandlungsvorgang deutlich zu erleichtern, ist wenigstens eine normal zur Anlenkachse 2 verlaufende Rückhaltefläche 8 vorgesehen. Dies bedeutet, dass die Anlenkachse 2 eine Flächennormale der Rückhaltefläche 8 bildet.

Grundsätzlich kann eine Arbeitserleichterung bereits dadurch erzielt werden, dass an einen Klemmabschnitt 6 in Öffnungsrichtung eine Rückhaltefläche 8 ansetzt. Eine bessere Abschirmung des sich zwischen den beiden Klemmanschnitten 6 ergebenden Behandlungsbereiches 9 ergibt sich allerdings, wenn an zwei einander gegenüberliegende Klemmabschnitte 6 beider Maulteile 5 jeweils in Öffnungsrichtung der erfindungsgemäßen Klemme 1 eine normal zur Anlenkachse 2 verlaufende Rückhaltefläche 8 ansetzt. Dadurch ergeben beide Rückhalteflächen 8 in Klemmstellung eine Art Schild, der das zwischen den Klemmabschnitten 6 im Behandlungsbereich 9 aufgespannte Gewebeteil vollständig vom umliegenden Gewebe einer Seite der Klemme 1 abschirmt.

Dadurch, dass die Rückhaltefläche 8 auf der dem Klemmabschnitt 6 gegenüberliegenden Seite einen ovalen Endabschnitt 10 aufweist, der keine spitzen Ecken ausbildet, kann das Verletzungsrisiko der durch die Rückhalteflächen 8 abgestützten Gewebeteile weiter reduziert werden. Zu diesem Zweck können darüber hinaus sämtliche Kanten der medizinischen Klemme entgratet sein.

Um eine punktuelle Druckbelastung und dadurch eventuell auftretende Schädigungen des umliegenden Gewebes zu vermeiden, kann der wenigstens eine Klemmabschnitt 6 eines Maulteiles 5 auf seiner dem zweiten Klemmabschnitt 6 dieses Maulteiles 5 gegenüberliegenden Seite bündig mit der Rückhaltefläche 8 abschließen.

Für verbesserte Behandlungsbedingungen kann es sich zudem als sinnvoll erweisen, wenn die erfindungsgemäße Klemme 1 in Klemmstellung in Ihrer Position fixiert wird. Dies kann durch zwei gegenläufige Zahn- oder Sperrstangen 11, die jeweils an beiden Griffteilen 4 ausgebildet sind, realisiert werden.

## Patentansprüche

1. Medizinische Klemme (1) mit zwei um eine Anlenkachse (2) drehbar miteinander verbundenen, gekreuzten Armen (3), die je einen Griffteil (4) und ein dem Griffteil (4) gegenüberliegendes Maulteil (5) aufweisen, **dadurch gekennzeichnet, dass** die Maulteile (5) je zwei in Richtung der Anlenkachse (2) voneinander beabstandete Klemmabschnitte (6) aufweisen.

2. Medizinische Klemme (1) nach Anspruch 1, **dadurch gekennzeichnet, dass** die Klemmabschnitte (6) in Schließrichtung atraumatische, zueinander versetzte und in Längsrichtung der Klemmabschnitte (6) verlaufende Klemmflächen (7) besitzen.

3. Medizinische Klemme (1) nach Anspruch 1 oder 2, **dadurch gekennzeichnet, dass** wenigstens an einen Klemmabschnitt (6) in Öffnungsrichtung der Klemme (1) eine normal zur Anlenkachse (2) verlaufende Rückhaltefläche (8) ansetzt.

4. Medizinische Klemme (1) nach Anspruch 3, **dadurch gekennzeichnet, dass** an zwei einander gegenüberliegende Klemmabschnitten (6) beider Maulteile (5) jeweils in Öffnungsrichtung der Klemme (1) eine normal zur Anlenkachse (2) verlaufende Rückhaltefläche (8) ansetzt.

5. Medizinische Klemme (1) nach Anspruch 4, **dadurch gekennzeichnet, dass** die Rückhaltefläche (8) auf der dem Klemmabschnitt (6) gegenüberliegenden Seite einen ovalen Endabschnitt (10) aufweist.

6. Medizinische Klemme (1) nach einem der Ansprüche 3 bis 5, **dadurch gekennzeichnet, dass** der wenigstens eine Klemmabschnitt (6) eines Maulteiles (5) auf seiner dem zweiten Klemmabschnitt (6) dieses Maulteiles (5) gegenüberliegenden Seite bündig mit der Rückhaltefläche (8) abschließt.

7. Medizinische Klemme (1) nach einem der vorangegangenen Ansprüche, **dadurch gekennzeichnet, dass** die Griffteile (4) zwei gegenläufige Sperrstangen (11) aufweisen.
